# EUROPÄISCHE PATENTSCHRIFT

(11) **EP 2 780 109 B1**
(45) Veröffentlichungstag und Bekanntmachung des Hinweises auf die Patenterteilung: **13.04.2016**
(21) Anmeldenummer: 12784011.4
(22) Anmeldetag: 13.11.2012
(51) Int. Cl.: B01J 23/14, B01J 23/835, B01J 37/02, C07C 211/00, C07C 213/00

(54) **VERFAHREN ZUR HERSTELLUNG VON SN-HALTIGEN KATALYSATOREN**
PROCESS FOR PRODUCING SN-CONTAINING CATALYSTS
PROCÉDÉ POUR LA PRODUCTION DES CATALYSEURS CONTENANT SN

(30) Priorität: 17.11.2011 EP 11189565
(43) Veröffentlichungstag der Anmeldung: 24.09.2014
(73) Patentinhaber: BASF SE, 67056 Ludwigshafen am Rhein (DE)
(72) Erfinder: HEIDEMANN, Thomas, 68519 Viernheim (DE); KUBANEK, Petr, 68163 Mannheim (DE); COELHO TSOU, Joana, 1031 KC Amsterdam (BE); BAAS, Heiko, 67149 Meckenheim (DE)
(74) Vertreter: BASF IP Association
(86) Internationale Anmeldenummer: PCT/EP2012/072438
(87) Internationale Veröffentlichungsnummer: WO 2013/072289

(56) Entgegenhaltungen:
- EP-A1- 0 224 947
- WO-A1-2006/078240
- WO-A1-2011/067199
- WO-A1-2011/094713

## Beschreibung

Die vorliegende Erfindung betrifft ein Verfahren zur Herstellung eines geträgerten zinnhaltigen (Sn-haltigen) Katalysators unter Verwendung einer Lösung (L) enthaltend Zinnnitrat und mindestens einen Komplexbildner, wobei die Lösung (L) keinen Feststoff oder nur einen sehr geringen Feststoffanteil aufweist. Weiterhin betrifft die vorliegende Erfindung den geträgerten zinnhaltigen Katalysator als solchen sowie die Verwendung dieses geträgerten zinnhaltigen Katalysators im Verfahren zur Herstellung von Aminen.

WO 2011/067199 betrifft aluminiumoxid-, zinn-, kupfer-, nickel- und kobalthaltige Katalysatoren und ein Verfahren zur Herstellung eines Amins durch Umsetzung eines primären oder sekundären Alkohols, Aldehyds und/oder Ketons mit Wasserstoff und einer Stickstoffverbindung, ausgewählt aus der Gruppe Ammoniak, primäre und sekundäre Amine, in Gegenwart dieses aluminiumoxid-, zinn-, kupfer-, nickel- und kobalthaltigen Katalysators.

Die in den Katalysatoren gemäß WO 2011/067199 enthaltenen Metallkomponenten, insbesondere Zinn (Sn), Kupfer (Cu), Kobalt (Co), Nickel (Ni), werden bei der Katalysatorherstellung zunächst in Salzform eingesetzt und durch einen Fällungsprozess auf den Katalysatorträger (vorzugsweise Aluminiumoxid) im Verfahren gemäß WO 2011/067199 aufgebracht. Während Nickel, Kobalt und Kupfer in Form ihrer Nitrate eingesetzt werden können, kommt Zinn nur in Form von Zinnchlorid zum Einsatz. Die Verwendung von Zinnnitrat ist in WO 2011/067199 bei der Katalysatorherstellung nicht offenbart und unter den dort beschriebenen Katalysatorherstellungsbedingungen auch nicht möglich, weil Zinnnitrat-Lösungen metastabil sind und Zinn aus solchen Lösungen sehr schnell auszufallen beginnt. Die in WO 2011/067199 zur Katalysatorherstellung beschriebenen Zinnchlorid-Lösungen sind jedoch mit dem Nachteil verbunden, dass das Chlorid bzw. das daraus während oder im Anschluss an die Zinnausfällung gebildete Chlor bereits in Spuren hochkorrosiv ist, was zu Korrosionsproblemen bei dem Herstellungsprozess des Katalysators sowie bei der späteren Katalysatoranwendung führt. Beispielsweise kann es dann zu Lochkorrosion oder Lochfraß kommen.

EP 0 224 947 A1, WO2006/078240 A1 und WO 20011/094713 A1 beschreiben weitere Sn-haltigen Katalysatoren. In der wissenschaftlichen Literatur ist es bereits bekannt, dass Zinn ausgehend von Zinnchlorid mit Citronensäure oder Tartronsäure auch Komplexe ausbilden kann. So offenbart beispielsweise P. R. Deacon et al., J. Chem. Soc. Dalton Trans. 1997, Seiten 3705 bis 3712, sowie T. D. Smith, J. Chem. Soc. 1965, Seiten 2145 bis 2149, die Herstellung solcher Zinnkomplexe sowie deren spektroskopische Charakterisierung. Der Einsatz von Zinnnitrat bei der Herstellung der Zinnkomplexe sowie deren Verwendung zur Herstellung von Katalysatoren oder die Verwendung solcher Katalysatoren zur Herstellung von Aminen ist jedoch in diesen Dokumenten nirgendwo offenbart.

Die der vorliegenden Erfindung zugrunde liegende Aufgabe besteht in der Bereitstellung eines neuen Verfahrens zur Herstellung von zinnhaltigen Katalysatoren und/oder den zinnhaltigen Katalysatoren als solchen.

Gelöst wird die Aufgabe durch das erfindungsgemäße Verfahren zur Herstellung eines geträgerten zinnhaltigen Katalysators, dadurch gekennzeichnet, dass eine Lösung (L) enthaltend Zinnnitrat und mindestens einen Komplexbildner auf den Träger aufgebracht wird, wobei die Lösung (L) keinen Feststoff oder einen Feststoffanteil von maximal 0,5 Gew.-% bezogen auf die Gesamtmenge an gelösten Komponenten enthält.

Durch das erfindungsgemäße Verfahren kann in einfacher und vorteilhafter Weise Zinn als Metallkomponente in einen Katalysator eingeführt bzw. auf den Katalysatorträger aufgebracht werden. Die eingesetzten Zinnnitrat-Lösungen mit Komplexbildner zeichnen sich durch eine hohe Stabilität aus, die über einen sehr langen Zeitraum anhält. Die Stabilität zeigt sich dadurch, dass diese Lösungen (L) klar bleiben und sich kein oder nur ein mengenmäßig sehr geringer Niederschlag und/oder Trübung in Form von ausgefälltem Zinn bildet. Die Lösung (L) enthält also keinen Feststoff oder einen Feststoffanteil in sehr geringen Mengen.

Aufgrund des Zusatzes von mindestens einem Komplexbildner zu der Lösung (L) enthaltend Zinnnitrat wird zudem gewährleistet, dass die Zinnnitratlösung auch bei Raumtemperatur oder höheren Temperaturen stabil ist. Zinnnitratlösungen ohne Komplexbildner sind bei Raumtemperatur oder höheren Temperaturen metastabil oder instabil, da sie schnell aufgrund von ausfallendem Zinn trübe werden. Die Stabilität von Zinnnitratlösungen ohne Komplexbildner, insbesondere wässrige Zinnnitratlösungen, kann ansonsten nur erhöht werden, wenn diese Lösungen bei sehr niedrigen Temperaturen, in der Regel kleiner 0 °C oder bevorzugt kleiner -10 °C durchgeführt werden. Auf diese Weise lassen sich zwar stabilere Zinnnitratlösungen herstellen. Da die weiteren Katalysatorherstellungsschritte jedoch mit höheren Temperaturen verbunden sind, ist es technisch sehr aufwändig, stark gekühlte Zinnnitratlösungen ohne Komplexbildner einzusetzen. Durch die Zugabe von mindestens einem Komplexbildner können stabile Zinnnitratlösungen unter technisch relevanten Bedingungen, also auch bei Raumtemperatur und darüber, in vorteilhafter Weise mehrere Tage bis zu mehreren Wochen aufbewahrt sowie zur Katalysatorherstellung eingesetzt werden.

Auf diese Weise hergestellte Katalysatoren zeichnen sich dadurch aus, dass das eingesetzte Zinn stabil und vollständig auf den Katalysatorträger gebunden ist. Die erfindungsgemäßen Katalysatoren sind zudem selektiver als Katalysatoren, die halogenhaltig (insbesondere chlorhaltig) sind und/oder bei denen eine metastabile Zinnnitrat-Lösung (ohne Komplexbildner) eingesetzt wird. Dieser Selektivitätsgewinn ist insbesondere bei großtechnischen Verfahren/Anlagen von entscheidender Bedeutung, weil dort bereits kleinere Selektivitätszunahmen große Kostenvorteile bewirken.

Eine vorzeitige Ausfällung von Zinn durch mangelnde Kühlung oder fehlenden Komplexbildnerzusatz von herkömmlichen Zinnitratlösungen vor der Weiterverarbeitung zur Katalysatorherstellung wirkt sich negativ auf die Leistungsfähigkeit des späteren Katalysators aus. Vermutlich wird die Dispersität des Zinns bzw. die Wechselwirkung mit den (gegebenenfalls vorhandenen) anderen Aktivkomponenten im späteren Katalysator negativ beeinflusst, wenn das Zinnnitrat nicht in Form einer stabilen Lösung bereitgestellt wird.

Infolge der Verwendung von Zinnnitrat anstelle von Zinnchlorid kommt es auch zu keinen Korrosionsproblemen bei der Katalysatorherstellung beziehungsweise der Verwendung dieser Katalysatoren zur Herstellung von Aminen durch im Katalysator verbleibende Chlorid- bzw. Chlorreste. So kann in den verwendeten Anlagen insbesondere das Auftreten von Lochkorrosion oder Lochfraß unterbunden werden.

Nachfolgend wird die vorliegende Erfindung weiter präzisiert.

Die zur Herstellung des geträgerten zinnhaltigen Katalysators eingesetzte Lösung (L) enthält Zinnnitrat und mindestens einen Komplexbildner. Die Lösung (L) enthält keinen Festststoff oder einen Feststoffanteil von maximal 0,5 Gew.-% bezogen auf die Gesamtmenge an gelösten Komponenten. Sofern die Lösung (L) einen Feststoffanteil aufweist, beträgt dieser vorzugsweise maximal 0,1 Gew.-%, insbesondere maximal 0,01 Gew.-%, bezogen auf die Gesamtmenge an gelösten Komponenten. Wie vorstehend bereits ausgeführt, handelt es sich bei der Lösung (L) um eine stabile, insbesondere klare Zinnnitratlösung, weil sie keinen Feststoff oder nur einen sehr geringen Feststoffanteil aufweist. Der Feststoff, sofern vorhanden, umfasst in der Regel eine oder mehrere der in der Lösung (L) enthaltenen Komponenten wie ausgefallenes Zinnnitrat, gegebenenfalls ausgefallene weitere Salze oder den ausgefallenen Komplexbildner sowie Gemische davon oder Reaktionsprodukte der einzelnen Komponenten.

Vorzugsweise ist die Lösung (L) stabil (klar) für einen Zeitraum von mindestens einem Tag bis maximal 30 Tage nach Herstellung der entsprechenden Lösung, insbesondere beträgt der Zeitrahmen mindestens fünf Tage bis maximal 15 Tage. Weiterhin ist es bevorzugt, dass die Lösung (L) stabil (klar) in einem Temperaturbereich von -20 °C bis maximal 120 °C ist, vorzugsweise von 10 °C bis 70 °C, besonders bevorzugt bei Raumtemperatur (20 bis 25 °C).

Als Lösungsmittel für die Lösung (L) kann prinzipiell jedes dem Fachmann bekannte Lösungsmittel verwendet werden, das sich zur Lösung von Salzen, insbesondere von Zinnnitrat, und/oder von Komplexbildnern eignet. Vorzugsweise ist die Lösung (L) eine wässrige Lösung.

Zinnitrat als solches ist dem Fachmann bekannt. Vorzugsweise wird Zinnnitrat in situ hergestellt durch Lösen von alkalischem Zinn (Zinngranalien) in Salpetersäure. Weiterhin ist es bevorzugt, dass das Zinnnitrat bei tiefen Temperaturen, vorzugsweise < 0 °C, besonders bevorzugt < -10°C, hergestellt wird. Besonders bevorzugt wird dabei eine wässrige Zinnnitratlösung hergestellt, wobei der Wassergehalt durch Zugabe von Eis, gekühltem Wasser und/oder verdünnter Salpetersäure eingestellt wird.

Als Komplexbildner eignet sich prinzipiell jeder dem Fachmann bekannte Komplexbildner, der mit Zinn, vorzugsweise in der Oxidationsstufe +2, einen Komplex ausbilden kann. Gegebenenfalls können auch zwei oder mehr unterschiedliche Komplexbildner eingesetzt werden. Bevorzugte Komplexbildner sind ausgewählt aus mindestens zweizähnigen Hydroxycarbonsäuren, Dicarbonsäuren, Multicarbönsäuren, Aminocarbonsäuren oder den Salzen der vorgenannten Säuren.

Mehr bevorzugt ist der Komplexbildner ausgewählt aus Glykolsäure, Milchsäure, Hydracylsäure, Hydroxybuttersäure, Hydroxyvaleriansäure, Äpfelsäure, Mandelsäure, Citronensäure, Zuckersäuren, Tartronsäure, Weinsäure, Oxalsäure, Malonsäure, Maleinsäure, Bernsteinsäure, Glutarsäure, Adipinsäure, Glycin, Hippursäure, EDTA (Ethylendiamintetraessigsäure), Alanin, Valin, Leucin oder Isoleucin.

Besonders bevorzugt ist der Komplexbildner Citronensäure.

Weiterhin kann die Lösung (L) weitere Komponenten enthalten. Bei diesen weiteren Komponenten handelt es sich vorzugsweise um Metallsalze, deren Metallkomponente ebenfalls in den geträgerten zinnhaltigen Katalysator eingebracht werden soll. Vorzugsweise ist mindestens ein Metallsalz ausgewählt aus einem Nickelsalz, einem Kobaltsalz und/oder einem Kupfersalz. Gegebenenfalls können auch weitere Salze verwendet werden, beispielsweise Rheniumsalze, Rutheniumsalze, Eisensalze oder Zinksalze. Weiterhin ist es bevorzugt, dass das Metallsalz und/oder die Lösung (L) kein Halogenid, insbesondere kein Chlorid enthält. Mehr bevorzugt werden als weitere Metallsalze die entsprechenden Nitrate der vorgenannten Metallsalze eingesetzt.

Besonders bevorzugt ist das weitere Metallsalz mindestens ein Metallsalz ausgewählt aus Nickelnitrat, Kobaltnitrat und/oder Kupfernitrat.

In der Lösung (L) können die einzelnen Komponenten in beliebigen Verhältnissen zueinander vorliegen. Vorzugsweise liegen Zinnnitrat sowie die gegebenenfalls vorhandenen weiteren Metallsalze zu 1 bis 50 Gew.-%, bevorzugt zu 5 bis 20 Gew.-%, in der Lösung (L), insbesondere in Wasser, vor (die Gewichtsangaben beziehen sich auf die Summe aller Salze relativ zum Gesamtgewicht der Lösung (L)). Vorzugsweise beträgt in der Lösung (L) das molare Verhältnis von Komplexbildner zu Zinn mindestens 1,5: 1, besonders bevorzugt 2 : 1 (Zinn liegt dabei als Zinnnitrat vor). Insbesondere beträgt das molare Verhältnis von Citronensäure zu Zinn 2:1.

In einer bevorzugten Ausführungsform der vorliegenden Erfindung enthält die Lösung (L) Zinnnitrat, Nickelnitrat, Kobaltnitrat, Kupfernitrat und Citronensäure.

Die Lösung (L) kann mit den dem Fachmann bekannten Verfahren hergestellt werden, beispielsweise durch sukzessive Zugabe der einzelnen Komponenten, Vorabmischungen von mindestens zwei Komponenten oder durch gleichzeitiges Mischen aller Komponenten. Vorzugsweise wird die Lösung (L) hergestellt durch i) Auflösen von Zinn in Salpetersäure bei einer Temperatur von maximal 5 °C und anschließender Zugabe von mindestens einem Komplexbildner, oder ii) durch Auflösen von mindestens einem Komplexbildner in Salpetersäure bei einer Temperatur von 0 °C bis 50 °C, vorzugsweise bei Raumtemperatur, und anschließender Zugabe von Zinn.

Erfindungsgemäß wird der geträgerte zinnhaltige Katalysator hergestellt, indem die vorstehend beschriebene Lösung (L) nach dem Fachmann bekannten Methoden auf den Katalysatorträger aufgebracht wird. Gegebenenfalls kann der Katalysatorträger bereits eine oder mehrere metallaktive Komponenten enthalten. Als Träger (Trägermaterial) kommen prinzipiell alle dem Fachmann bekannten Träger in Frage. Vorzugsweise ist der Träger Aluminiumoxid. Gegebenenfalls können auch Gemische aus Aluminiumoxid und anderen Trägermaterialien eingesetzt werden, beispielsweise siliciumhaltige und/oder zirkoniumhaltige Träger. Weitere geeignete Träger, Verfahren zum Aufbringen von salzhaltigen Lösungen sowie weitere Verfahrensschritte zur Herstellung eines geträgerten Katalysators als solchen sind beispielsweise in WO 2011/067199 offenbart.

Vorzugsweise erfolgt das Aufbringen der Lösung (L) auf den Katalysatorträger durch ein Fällungsverfahren, wie es beispielsweise in WO 2011/067199 beschrieben ist. In diesem Fällungsverfahren wird das Zinnnitrat, mindestens ein Komplexbildner und gegebenenfalls mindestens ein weiteres Metallsalz durch Zugabe von mindestens einer Base (B) aus der Lösung (L) auf den Träger ausgefällt.

Vorzugsweise wird die Base (B) als wässrige Lösung zugegeben. Es ist bevorzugt, dass die Base (B) ausgewählt ist aus Natriumcarbonat, Natriumhydroxid, Kaliumcarbonat, Kaliumhydroxid oder alkalimetallfreien Basen wie Ammoniak, Ammoniumcarbonat, Ammoniumhydrogencarbonat, Ammoniumcarbamat, Ammoniumoxalat, Ammoniummalonat, Urotropin oder Harnstoff, vorzugsweise ist die Base (B) Natriumcarbonat.

Beispielsweise können im erfindungsgemäßen Verfahren die erfindungsgemäßen Katalysatoren über eine gemeinsame Fällung (Mischfällung) aller ihrer Komponenten hergestellt werden. Dazu wird zweckmäßigerweise eine die Katalysatorkomponenten enthaltende wässrige Salzlösung in der Wärme und unter Rühren so lange mit einer wässrigen Base - beispielsweise Natriumcarbonat, Natriumhydroxid, Kaliumcarbonat oder Kaliumhydroxid - versetzt, bis die Fällung vollständig ist. Es kann auch mit alkalimetallfreien Basen wie Ammoniak, Ammoniumcarbonat, Ammoniumhydrogencarbonat, Ammoniumcarbamat, Ammoniumoxalat, Ammoniummalonat, Urotropin, Harnstoff etc. gearbeitet werden. Die Art der verwendeten Salze ist im Allgemeinen nicht kritisch: Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen -verursachen oder indem sie durch Komplexbildung die Fällung erschweren oder verhindern.

Die bei diesen Fällungsreaktionen erhaltenen Niederschläge sind im Allgemeinen chemisch uneinheitlich und bestehen u. a. aus Mischungen der Oxide, Oxidhydrate, Hydroxide, Carbonate und unlöslichen und basischen Salzen der eingesetzten Metalle. Es kann sich für die Filtrierbarkeit der Niederschläge als günstig erweisen, wenn sie gealtert werden, das heißt wenn man sie noch einige Zeit nach der Fällung, gegebenenfalls in Wärme oder unter Durchleiten von Luft, sich selbst überlässt.

Die nach diesen Fällungsverfahren erhaltenen Niederschläge können wie üblich weiterverarbeitet werden. Zunächst werden die Niederschläge gewaschen. Über die Dauer des Waschvorgangs und über die Temperatur und Menge des Waschwassers kann der Gehalt an Alkalimetall, das durch die als Fällungsmittel eventuell verwendete (Mineral)base zugeführt wurde, beeinflusst werden. Im Allgemeinen wird durch Verlängerung der Waschzeit oder Erhöhung der Temperatur des Waschwassers der Gehalt an Alkalimetall abnehmen. Nach dem Waschen wird das Fällgut im Allgemeinen bei 80 bis 200 °C, vorzugsweise bei 100 bis 150 °C, getrocknet und danach calciniert.

Die Calcinierung (Calcinationsschritt) wird im Allgemeinen bei Temperaturen zwischen 300 und 800 °C, vorzugsweise bei 400 bis 600 °C, insbesondere bei 420 bis 550 °C ausgeführt.

Weiterhin ist es bevorzugt, dass nach Aufbringen der Lösung (L) auf den Träger mindestens ein weiterer Schritt ausgewählt aus einem Trocknungsschritt, einem Calcinationsschritt, einem Konditionierungsschritt, einem Formgebungsschritt, einem Reduktionsschritt oder einem Passivierungsschritt durchgeführt wird.

Die konkrete Durchführung der obenstehend beschriebenen weiteren Schritte ist beispielsweise in WO 2011/067199 offenbart. Konkrete Temperaturbereiche für den Trocknungsschritt sowie den Calcinierungsschritt sind zudem im vorstehenden Text im Rahmen des beispielhaft aufgeführten Fällungsverfahrens aufgeführt.

In einer bevorzugten Ausführungsform der vorliegenden Erfindung wird der Komplexbildner während oder im Anschluss an den Calcinationsschritt aus dem geträgerten zinnhaltigen Katalysator entfernt. Besonders bevorzugt erfolgt dies, indem der Komplexbildner während des Calcinationsschrittes durch oxidativen Abbrand aus dem geträgerten, zinnhaltigen Katalysator entfernt wird. Vorzugsweise wird der Komplexbildner rückstandsfrei (vollständig) im Rahmen des Calcinationsschrittes aus dem Katalysator entfernt.

Ein weiterer Gegenstand der vorliegenden Erfindung ist ein geträgerter zinnhaltiger Katalysator als solcher, herstellbar nach dem vorstehend beschriebenen Verfahren.

Vorzugsweise enthält der erfindungsmäßige geträgerte zinnhaltige Katalysator 0,2 bis 5 Gew.-% Zinn, berechnet als SnO. Weiterhin ist es bevorzugt, dass der erfindungsgemäße Katalysator mindestens eine zusätzliche Metallkomponente, ausgewählt aus Kupfer, Nickel und/oder Kobalt enthält. Ebenfalls ist es bevorzugt, dass der Katalysatorträger Aluminiumoxid ist.

Die erfindungsgemäßen Katalysatoren werden bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten. In diesem Zusammenhang wird das oxidische Trägermaterial, vorzugsweise Aluminiumoxid (Al₂O₃), als zur katalytisch aktiven Masse gehörig gewertet.

Die Katalysatoren werden dergestalt eingesetzt, dass man die katalytisch aktive, zu Pulver vermahlene Masse in das Reaktionsgefäß einbringt oder dass man die katalytisch aktive Masse nach Mahlung, Vermischung mit Formhilfsmitteln, Formung und Temperung als Katalysatorformkörper - beispielsweise als Tabletten, Kugeln, Ringe, Extrudate (z. B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und der oben genannten Katalysatorträgermaterialien definiert und enthält im Wesentlichen die folgenden Bestandteile: Aluminiumoxid (Al₂O₃), sauerstoffhaltige Verbindungen des Zinns und gegebenenfalls sauerstoffhaltige Verbindungen des Kupfers, Nickels und/oder Kobalts.

Die Summe der oben genannten Bestandteile der katalytisch aktiven Masse beträgt üblicherweise 70 bis 100 Gew.-%, bevorzugt 80 bis 100 Gew.-%, mehr bevorzugt 90 bis 100 Gew.-%, noch mehr bevorzugt > 95 Gew.-% bis 100 Gew.-%, besonders bevorzugt > 98 Gew.-% bis 100 Gew.-%, insbesondere > 99 Gew.-% bis 100 Gew.-%, z. B. genau 100 Gew.-%.

Die katalytisch aktive Masse der erfindungsgemäßen und im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen IA bis VI A und I B bis VII B und VIII des Periodensystems, enthalten.

Beispiele für solche Elemente bzw. deren Verbindungen sind: Übergangsmetalle, wie Mn bzw. MnO₂, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. CeO₂ oder Pr bzw. Pr₂O₃; Erdalkalimetalloxide, wie SrO; Erdalkalimetallcarbonate, wie MgCO₃, CaCO₃ und BaCO₃; Boroxid (B₂O₃).

Die katalytisch aktive Masse des Katalysators enthält vorzugsweise vor dessen Reduktion mit Wasserstoff im Bereich von 0,2 bis 5,0 Gew.-%, besonders im Bereich von 0,4 bis 4,0 Gew-%, weiter besonders im Bereich von 0,6 bis 3,0 Gew.-%, weiter besonders bevorzugt im Bereich von 0,7 bis 2,5 Gew.-%, sauerstoffhaltige Verbindungen des Zinns, berechnet als SnO.

Die katalytisch aktive Masse des Katalysators enthält gegebenenfalls vor dessen Reduktion mit Wasserstoff bevorzugt im Bereich von 5,0 bis 35 Gew.-%, besonders im Bereich von 10 bis 30 Gew.-%, weiter besonders im Bereich von 12 bis 28 Gew.-%, ganz besonders 15 bis 25 Gew.-%, sauerstoffhaltige Verbindungen des Kobalts, berechnet als CoO.

Die katalytisch aktive Masse des Katalysators enthält gegebenenfalls in einer Ausführungsform der vorliegenden Erfindung vor dessen Reduktion mit Wasserstoff weiterhin bevorzugt im Bereich von 15 bis 80 Gew. %, besonders 30 bis 70 Gew.-%, weiter besonders 35 bis 65 Gew.-%, sauerstoffhaltige Verbindungen des Aluminiums, berechnet als Al₂O₃, 1 bis 20 Gew.-%, besonders 2 bis 18 Gew. %, weiter besonders 5 bis 15 Gew. %, sauerstoffhaltige Verbindungen des Kupfers, berechnet als CuO, und 5 bis 35 Gew.-%, besonders 10 bis 30 Gew.-%, weiter besonders 12 bis 28 Gew.-%, ganz besonders 15 bis 25 Gew.-%, sauerstoffhaltige Verbindungen des Nickels, berechnet als NiO.

Sofern vorhanden, ist das Molverhältnis von Nickel zu Kupfer bevorzugt größer 1, besonders bevorzugt größer 1,2, weiter besonders bevorzugt im Bereich von 1,8 bis 8,5.

Die BET-Oberfläche (ISO 9277:1995) der erfindungsgemäßen und im erfindungsgemäßen Verfahren eingesetzten Katalysatoren liegt bevorzugt im Bereich von 30 bis 250 m²/g, besonders im Bereich von 90 bis 200 m²/g, weiter besonders im Bereich von 130 bis 190m²/g. Diese Bereiche werden insbesondere durch Calciniertemperaturen bei der Katalysatorherstellung im Bereich von 400 bis 600 °C, besonders 420 bis 550 °C, erzielt.

In einer bevorzugten Ausführungsform der vorliegenden Erfindung, enthält der erfindungsgemäße Katalysator
i) 0,2 bis 5 Gew.-% Zinn,
ii) 15 bis 80 Gew.-% Aluminium,
iii) 1 bis 20 Gew.-% Kupfer,
iv) 5 bis 35 Gew.-% Nickel, und
v) 5 bis 35 Gew.-% Cobalt,
wobei die Gewichtsangaben der Komponenten i) bis v) als Oxide nach einem Calcinationsschritt und vor einem Reduktionsschritt mit Wasserstoff bestimmt werden.

Nach einer weiteren bevorzugten Ausführungsform der vorliegenden Erfindung enthält der erfindungsgemäße Katalysator kein Halogen (beispielsweise in Form von Halogenid), insbesondere kein Chlor (beispielsweise in Form von Chloriden oder sonstigen chlorhaltigen Verbindungen).

Ein weiterer Gegenstand der vorliegenden Erfindung ist ein Verfahren zur Herstellung eines Amins (A) in Gegenwart eines erfindungsgemäßen Katalysators gemäß den vorstehenden Ausführungen. Verfahren zur Herstellung von Aminen sind dem Fachmann prinzipiell bekannt. Im nachfolgenden Text werden zunächst die allgemeinen Verfahrensbedingungen wie Druck, Temperatur, Eduktverhältnisse etc. beschrieben. Im Anschluss daran folgt die Beschreibung der konkret einsetzbaren Edukte wie ein primärer Alkohol oder eine stickstoffhaltige Verbindung (S) sowie das im erfindungsgemäßen Verfahren erhaltene Produkt (Amin (A)). Die als Edukt eingesetzte stickstoffhaltige Verbindung (S) kann auch als Aminierungsmittel oder Aminkomponente bezeichnet werden. Unter Aminierung wird die Umsetzung einer solchen stickstoffhaltigen Verbindung (S) mit einem weiteren Edukt wie einem primären Alkohol oder einem Aldehyd zum Amin (A) verstanden.

Das erfindungsgemäße Verfahren zur Herstellung des Amins (A) kann kontinuierlich oder diskontinuierlich durchgeführt werden. Bevorzugt ist eine kontinuierliche Fahrweise.

Für die Synthese in der Gasphase werden die Edukte gezielt, bevorzugt in einem Kreisgasstrom, verdampft und gasförmig dem Reaktor zugeführt. Geeignete Amine für eine Gasphasensynthese sind Amine, die aufgrund ihrer Siedepunkte und der Siedepunkte ihrer Edukte verfahrenstechnisch im Rahmen der Prozessparameter in der Gasphase gehalten werden können. Das Kreisgas dient zum einen der Verdampfung der Edukte und zum anderen als Reaktionspartner für die Aminierung.

In der Kreisgasfahrweise werden die Ausgangsstoffe (Alkohol, Aldehyd und/oder Keton, Wasserstoff und die Stickstoffverbindung) in einem Kreisgasstrom verdampft und gasförmig dem Reaktor zugeführt.

Die Edukte (Alkohol, Aldehyd und/oder Keton, Wasserstoff und die Stickstoffverbindung) können auch als wässrige Lösungen verdampft und mit dem Kreisgasstrom auf das Katalysatorbett geleitet werden.

Bevorzugte Reaktoren sind Rohrreaktoren. Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors".

Alternativ erfolgt die Umsetzung vorteilhaft in einem Rohrbündelreaktor oder in einer Monostranganlage.

Bei einer Monostranganlage kann der Rohrreaktor, in dem die Umsetzung erfolgt, aus einer Hintereinanderschaltung mehrerer (z. B. zweier oder dreier) einzelner Rohrreaktoren bestehen. Optional ist hier vorteilhaft eine Zwischeneinspeisung von Feed (enthaltend das Edukt und/oder Ammoniak und/oder H₂) und/oder Kreisgas und/oder Reaktoraustrag aus einem nachgeschalteten Reaktor möglich.

Die Kreisgasmenge liegt bevorzugt im Bereich von 40 bis 1500 m³ (bei Betriebsdruck) / [m³ Katalysator (Schüttvolumen) • h], insbesondere im Bereich von 100 bis 700 m³ (bei Betriebsdruck) / [m³ Katalysator (Schüttvolumen) • h].

Das Kreisgas enthält bevorzugt mindestens 10, besonders 50 bis 100, ganz besonders 80 bis 100 Vol.-% H₂.

Für die Synthese in der Flüssigphase sind alle Edukte und Produkte geeignet, welche schwer verdampfbar oder thermisch labil sind. In diesen Fällen kommt als weiterer Vorteil hinzu, dass auf eine Verdampfung und Rekondensation des Amins im Prozess verzichtet werden kann.

Das erfindungsgemäße Verfahren zur Herstellung eines Amins (A) wird bevorzugt kontinuierlich durchgeführt, wobei der vorstehend beschriebene Katalysator bevorzugt als Festbett im Reaktor angeordnet ist. Dabei ist sowohl eine Anströmung des Katalysatorfestbetts von oben als auch von unten möglich. Der Gasstrom wird dabei durch Temperatur, Druck und Menge so eingestellt, dass auch schwerer siedende (hoch siedende) Reaktionsprodukte in der Gasphase verbleiben.

Die stickstoffhaltige Verbindung (S) kann bezüglich der zu aminierenden Verbindung mit mindestens einer alkoholischen Hydroxylgruppe bzw. einer Aldehydgruppe bzw. einer Ketogruppe in stöchiometrischen, unter- oder überstöchiometrischen Mengen eingesetzt werden.

Bevorzugt wird im Falle der Aminierung von Alkoholen, Aldehyden oder Ketonen mit primären oder sekundären Aminen das Amin in ca. stöchiometrischer Menge oder geringfügig überstöchiometrischer Menge pro Mol zu aminierender alkoholischer Hydroxylgruppe, Aldehydgruppe oder Ketogruppe eingesetzt.

Die Aminkomponente (stickstoffhaltige Verbindung (S)) wird bevorzugt in der 0,90- bis 100-fachen molaren Menge, insbesondere in der 1,0- bis 10-fachen molaren Menge, jeweils bezogen auf den/das eingesetzte/n Alkohol, Aldehyd und/oder Keton eingesetzt.

Speziell Ammoniak (als stickstoffhaltige Verbindung (S)) wird im Allgemeinen mit einem 1,5 bis 250-fachen, bevorzugt 2- bis 100-fachen, insbesondere 2- bis 10-fachen molaren Überschuss pro Mol umzusetzender alkoholischer Hydroxylgruppe, Aldehydgruppe oder Ketogruppe eingesetzt.

Höhere Überschüsse sowohl an Ammoniak als auch an primären oder sekundären Aminen sind möglich.

Bevorzugt wird eine Abgasmenge von 5 bis 800 Normkubikmeter/h, insbesondere 20 bis 300 Normkubikmeter/h, gefahren. (Normkubikmeter = auf Normalbedingungen umgerechnetes Volumen).

Die Aminierung der primären oder sekundären Alkoholgruppen, Aldehydgruppen oder Ketogruppen des Edukts kann in der Flüssigphase oder in der Gasphase durchgeführt werden.

Beim Arbeiten in der Flüssigphase leitet man die Edukte (Alkohol, Aldehyd oder Keton plus Ammoniak oder Amin) simultan in flüssiger Phase bei Drücken von im Allgemeinen 5 bis 30 MPa (50-300 bar), bevorzugt 5 bis 25 MPa, besonders bevorzugt 15 bis 25 MPa, und Temperaturen von im Allgemeinen 80 bis 350 °C, besonders 100 bis 300 °C, bevorzugt 120 bis 270 °C, besonders bevorzugt 130 bis 250 °C, insbesondere 170 bis 230 °C, inklusive Wasserstoff über den Katalysator, der sich üblicherweise in einem bevorzugt von außen beheizten Festbettreaktor befindet. Es ist dabei sowohl eine Rieselfahrweise als auch eine Sumpffahrweise möglich. Die Katalysatorbelastung liegt im Allgemeinen im Bereich von 0,05 bis 5, bevorzugt 0,1 bis 2, besonders bevorzugt 0,2 bis 0,6 kg Alkohol, Aldehyd oder Keton pro Liter Katalysator (Schüttvolumen) und Stunde. Gegebenenfalls kann eine Verdünnung der Edukte mit einem geeigneten Lösungsmittel wie Tetrahydrofuran, Dioxan, N-Methylpyrrolidon oder Ethylenglykoldimethylether erfolgen. Es ist zweckmäßig, die Reaktanden bereits vor der Zuführung in das Reaktionsgefäß zu erwärmen, und zwar bevorzugt auf die Reaktionstemperatur.

Beim Arbeiten in der Gasphase werden die gasförmigen Edukte (Alkohol, Aldehyd oder Keton plus Ammoniak oder Amin) in einem zur Verdampfung ausreichend groß gewählten Gasstrom, bevorzugt Wasserstoff, bei Drücken von im Allgemeinen 0,1 bis 40 MPa (1 bis 400 bar), bevorzugt 0,1 bis 10 MPa, besonders bevorzugt 0,1 bis 5 MPa, in Gegenwart von Wasserstoff über den Katalysator geleitet. Die Temperaturen für die Aminierung von Alkoholen betragen im Allgemeinen 80 bis 350 °C, besonders 100 bis 300 °C, bevorzugt 120 bis 270 °C, besonders bevorzugt 160 bis 250 °C. Die Reaktionstemperaturen bei der hydrierenden Aminierung von Aldehyden und Ketonen betragen im Allgemeinen 80 bis 350 °C, besonders 90 bis 300 °C, bevorzugt 100 bis 250 °C. Es ist dabei sowohl eine Anströmung des Katalysatorfestbetts von oben als auch von unten möglich. Den erforderlichen Gasstrom erhält man bevorzugt durch eine Kreisgasfahrweise.

Die Katalysatorbelastung liegt im Allgemeinen im Bereich von 0,01 bis 2, bevorzugt 0,05 bis 0,5 kg Alkohol, Aldehyd oder Keton pro Liter Katalysator (schüttvolumen) und Stunde.

Der Wasserstoff wird der Reaktion im Allgemeinen in einer Menge von 5 bis 400 I, bevorzugt in einer Menge von 50 bis 2001 pro Mol Alkohol-, Aldehyd- oder Ketonkomponente zugeführt, wobei die Literangaben jeweils auf Normalbedingungen umgerechnet wurden (S.T.P.).

Die Aminierung von Aldehyden bzw. Ketonen unterscheidet sich in der Durchführung von der Aminierung von Alkoholen dadurch, dass bei der Aminierung von Aldehyden und Ketonen mindestens stöchiometrische Mengen an Wasserstoff vorhanden sein müssen.

Sowohl beim Arbeiten in der Flüssigphase als auch beim Arbeiten in der Gasphase ist die Anwendung höherer Temperaturen und höherer Gesamtdrücke und Katalysatorbelastungen möglich. Der Druck im Reaktionsgefäß, welcher sich aus der Summe der Partialdrücke des Aminierungsmittels, des Alkohols, Aldehyds bzw. Ketons und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl beim kontinuierlichen Arbeiten in der Flüssigphase als auch beim kontinuierlichen Arbeiten in der Gasphase kann das überschüssige Aminierungsmittel zusammen mit dem Wasserstoff im Kreis geführt werden.

Ist der Katalysator als Festbett angeordnet, kann es für die Selektivität der Reaktion vorteilhaft sein, die Katalysatorformkörper im Reaktor mit inerte Füllkörpern zu vermischen, sie sozusagen zu "verdünnen". Der Anteil der Füllkörper in solchen Katalysatorzubereitungen kann 20 bis 80, bevorzugt 30 bis 60 und besonders bevorzugt 40 bis 50 Volumenteile betragen.

Das im Zuge der Umsetzung gebildete Reaktionswasser (jeweils ein Mol pro Mol umgesetzte Alkoholgruppe, Aldehydgruppe bzw. Ketogruppe) wirkt sich im Allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Aus dem Reaktionsaustrag werden, nachdem dieser zweckmäßigerweise entspannt worden ist, der überschüssige Wasserstoff und das gegebenenfalls vorhandene überschüssige Aminierungsmittel entfernt und das erhaltene Reaktionsrohprodukt gereinigt, z. B. durch eine fraktionierende Rektifikation. Geeignete Aufarbeitungsverfahren sind z. B. in EP 1 312 600 A und EP 1 312 599 A (beide BASF AG) beschrieben. Das überschüssige Aminierungsmittel und der Wasserstoff werden vorteilhaft wieder in die Reaktionszone zurückgeführt. Das gleiche gilt für die eventuell nicht vollständig umgesetzte Alkohol-, Aldehyd- bzw. Ketonkomponente.

Unumgesetzte Edukte und gegebenenfalls anfallende geeignete Nebenprodukte können wieder in die Synthese zurückgeführt werden. Nicht umgesetzte Edukte können in diskontinuierlicher oder kontinuierlicher Fahrweise nach Kondensation der Produkte im Abscheider in dem Kreisgasstrom erneut über das Katalysatorbett geströmt werden.

Mit dem erfindungsgemäßen Verfahren können prinzipiell alle dem Fachmann bekannten Amine hergestellt werden. Sinngemäßes gilt für die bei dem Herstellungsverfahren eingesetzten Edukte. Als Edukte werden eine stickstoffhaltige Verbindung (S) als Aminierungsmittel sowie eine zu aminierende Verbindung wie ein primärer Alkohol oder ein Aldehyd eingesetzt. Darüber hinaus wird in der Regel Wasserstoff als Edukt verwendet. Geeignete Edukte sowie das bei diesem Verfahren gebildete Produkt (Amin (A)) sind beispielsweise in WO 2011/067199 offenbart.

Vorzugsweise wird das erfindungsgemäße Verfahren zur Herstellung eines Amins (A) durchgeführt, indem ein primärer Alkohol, ein sekundärer Alkohol, ein Aldehyd und/oder ein Keton mit Wasserstoff und einer stickstoffhaltigen Verbindung (S) umgesetzt werden. Die stickstoffhaltige Verbindung (S) ist vorzugsweise Ammoniak, ein primäres Amin und/oder ein sekundäres Amin. Weiterhin ist es bevorzugt, dass der primäre Alkohol, der sekundäre Alkohol, das Aldehyd, das Keton und/oder die stickstoffhaltige Verbindung (S) als wässrige Lösung eingesetzt werden.

Als Alkohole eignen sich unter den oben genannten Voraussetzungen praktisch alle primären und sekundären Alkohole mit aliphatischer OH-Funktion. Die Alkohole können geradkettig, verzweigt oder zyklisch sein. Sekundäre Alkohole werden ebenso aminiert wie primäre Alkohole. Die Alkohole können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen,
oder auch gegebenenfalls unter den Bedingungen der hydrierenden Aminierung hydriert werden, beispielsweise CC-Doppel- oder Dreifachbindungen. Sollen mehrwertige Alkohole, wie z. B. Diole oder Triole, besonders Glykole, aminiert werden, so hat man es über die Steuerung der Reaktionsbedingungen in der Hand, bevorzugt Aminoalkohole, zyklisch Amine oder mehrfach aminierte Produkte zu erhalten.

Die Aminierung von 1,2-Diolen führt je nach Wahl der Reaktionsbedingungen besonders zu 1-Amino-2-hydroxy- oder 1,2-Diamino-Verbindungen.

Die Aminierung von 1,4-Diolen führt je nach Wahl der Reaktionsbedingungen zu 1-Amino-4-hydroxy-, 1,4-Diamino-Verbindungen oder zu fünfgliedrigen Ringen mit einem Stickstoffatom (Pyrrolidine).

Die Aminierung von 1,6-Diolen führt je nach Wahl der Reaktionsbedingungen zu 1-Amino-6-hydroxy-, 1,6-Diamino-Verbindungen oder zu siebengliedrigen Ringen mit einem Stickstoffatom (Hexamethylenimine).

Die Aminierung von 1,5-Diolen führt je nach Wahl der Reaktionsbedingungen zu 1-Amino-5-hydroxy-, 1,5-Diamino-Verbindungen oder zu sechsgliedrigen Ringen mit einem Stickstoffatom (Piperidine, 1,5-Di-piperidinyl-pentane).

Aus Diglykol (DEG) kann demnach durch Aminierung mit NH₃ Monoaminodiglykol (= ADG = H₂N-CH₂CH₂-O-CH₂CH₂-OH), Diaminodiglykol (H₂N-CH₂CH₂-O-CH₂CH₂-NH₂) oder Morpholin erhalten werden. Besonders bevorzugt ist hier das ADG als Verfahrensprodukt.

Aus Diethanolamin wird entsprechend besonders bevorzugt Piperazin erhalten. Aus Triethanolamin kann N-(2-Hydroxyethyl)-piperazin erhalten werden.

Besonders bevorzugte Alkohole sind Methanol, Ethanol, n-Propanol, i-Propanol, n-Butanol, sek.-Butanol, 1,4-Butandiol, 1,5-Pentandiol, 1,6-Hexandiol, 2-Ethylhexanol, Cyclohexanol, Fettalkohole, Ethylenglykol, Diethylenglykol (DEG), Triethylenglykol (TEG), 2-(2-Dimethylamino-ethoxy)ethanol, N-Methyldiethanolamin - und 2-(2-Dimethylaminoethoxy)ethanol.

Als im erfindungsgemäßen Verfahren einsetzbare Ketone eignen sich unter den oben genannten Voraussetzungen praktisch alle aliphatischen und aromatischen Ketone. Die aliphatischen Ketone können geradkettig, verzweigt oder zyklisch sein, die Ketone können Heteroatome enthalten. Die Ketone können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beilspielsweise CC-Doppel- oder Dreifachbindungen. Sollen mehrwertige Ketone aminiert werden, so hat man es über die Steuerung der Reaktionsbedingungen in der Hand, Aminoketone, Aminoalkohole, cyclische Amine oder mehrfach aminierte Produkte zu erhalten.

Bevorzugt werden die folgenden Ketone aminierend hydriert:
Aceton, Ethylmethylketon, Methylvinylketon, Isobutylmethylketon, Butanon, 3-Methylbutan-2-on, Diethylketon, Tetraion, Acetophenon, p-Methyl-acetophenon, p-Methoxy-acetophenon, m-Methoxy-acetophenon, 1-Acetyl-naphthalin, 2-Acetyl-naphthalin, 1-Phenyl-3-butanon, Cyclobutanon, Cyclopentanon, Cyclopentenon, Cyclohexanon, Cyclohexenon, 2,6-Dimethylcyclohexanon, Cycloheptanon, Cyclododecanon, Acetylaceton, Methylglyoxal und Benzophenon.

Als im erfindungsgemäßen Verfahren einsetzbare Aldehyde eignen sich unter den oben genannten Voraussetzungen praktisch alle aliphatischen und aromatischen Aldehyde. Die aliphatischen Aldehyde können geradkettig, verzweigt oder zyklisch sein, die Aldehyde können Heteroatome enthalten. Die Aldehyde können ferner Substituenten tragen oder funktionelle Gruppen enthalten, welche sich unter den Bedingungen der hydrierenden Aminierung inert verhalten, beispielsweise Alkoxy-, Alkenyloxy-, Alkylamino- oder Dialkylaminogruppen, oder auch gegebenenfalls unter den Bedingungen der hydrierenden Aminierung hydriert werden, beispielsweise CC-Doppel- oder Dreifachbindungen. Sollen mehrwertige Aldehyde oder Ketoaldehyde aminiert werden, so hat man es über die Steuerung der Reaktionsbedingungen in der Hand, Aminoalkohole, cyclische Amine oder mehrfach aminierte Produkte zu erhalten.

Bevorzugt werden die folgenden Aldehyde aminierend hydriert:
Formaldehyd, Acetaldehyd, Propionaldehyd, n-Butyraldehyd, Isobutyraldehyd, Pivalinaldehyd, n-Pentanal, n-Hexanal, 2-Ethylhexanal, 2-Methylpentanal, 3-Methylpentanal, 4-Methylpentanal, Glyoxal, Benzaldehyd, p-Methoxybenzaldehyd, p-Methylbenzaldehyd, Phenylacetaldehyd, (p-Methoxy-phenyl)acetaldehyd, (3,4-Dimethoxyphenyl)-acetaldehyd, 4-Formyltetrahydropyran, 3- Formyltetrahydrofuran, 5-Formylvaleronitril, Citronellal, Lysmeral, Acrolein, Methacrolein, Ethylacrolein, Citral, Crotonaldehyd, 3-Methoxypropionaldehyd,3-Aminopropionaldehyd, Hydroxypivalinaldehyd, Dimethylolpropionaldehyd, Dimethylolbutyraldehyd, Furfural, Glyoxal, Glutaraldehyd sowie hydroformylierte Oligomere und Polymere, wie z. B. hydroformyliertes Polyisobuten (Polyisobutenaldehyd) oder durch Metathese von 1-Penten und Cyclopenten erhaltenes und hydroformyliertes Oligomer.

Bei Verwendung von Ammoniak als Aminierungsmittel wird die alkoholische Hydroxylgruppe bzw. die Aldehydgruppe bzw. die Ketogruppe zunächst in die primäre Aminogruppen (-NH₂) umgewandelt. Das so gebildete primäre Amin können mit weiterem Alkohol bzw. Aldehyd bzw. Keton zu dem entsprechenden sekundären Amin und diese wiederum mit weiterem Alkohol bzw. Aldehyd bzw. Keton zu dem entsprechenden, vorzugsweise symmetrischen, tertiären Amin reagieren. Je nach Zusammensetzung des Reaktionsansatzes oder des Eduktstroms (bei kontinuierlicher Fahrweise) und je nach den angewandten Reaktionsbedingungen - Druck, Temperatur, Reaktionszeit (Katalysatorbelastung) - lassen sich auf diese Weise je nach Wunsch bevorzugt primäre, sekundäre oder tertiäre Amine darstellen.

Mit dem erfindungsgemäßen Verfahren besonders bevorzugt hergestellte Amine sind zum Beispiel Morpholin (aus Monoaminodiglykol), Monoaminodiglykol, Morpholin und/oder 2,2'-Dimorpholinodiethylether (DMDEE) (aus DEG und Ammoniak), 6-Dimethylaminöhexanol-1 (aus Hexandiol und Dimethylamin (DMA)), Triethylamin (aus Ethanol und Diethylamin (DEA)), Dimethylethylamin (aus Ethanol und DMA), N-(C₁₋₄-alkyl)morpholin (aus DEG und Mono(C₁₋₄-alkyl)amin), N-(C₁₋₄-alkyl)piperidin (aus 1,5-Pentandiol und Mono(C₁₋₄-alkyl)amin), Piperazin und/oder Diethylentriamin (DETA) (aus N-(2-Aminoethyl)-ethanolamin (AEEA) und Ammoniak), N-Methylpiperazin (aus Diethanolamin und MMA), N,N'-Dimethylpiperazin (aus N-Methyldiethanolamin und MMA), 1,2-Ethylendiamin (EDA) und/oder Diethylentriamin (DETA) und/oder PIP (aus Monoethanolamin (MEOA) und Ammoniak), 2-Ethylhexylamin und Bis(2-Ethylhexyl)amin (aus 2-Ethylhexanol und NH₃), Tridecylamin und Bis(Tridecyl)amin (aus Tridecanol und NH₃), n-Octylamin (aus n-Octanol und NH₃), 1,2-Propylendiamin (aus 2-Hydroxy-propylamin und NH₃), 1-Diethylamino-4-aminopentan (aus 1-Diethylamino-4-hydroxypentan und NH₃), N,N-Di(C₁₋₄-alkyl)cyclohexylamin (aus Cyclohexanon und/oder Cyclohexanol und Di(C₁₋₄-alkyl)amin), z. B. N,N-Dimethyl-N-cyclohexylamin (DMCHA), Polyisobutenamin (PIBA; mit z. B. n∼1000) (aus Polyisobutenaldehyd und NH₃), N,N-Diisopropyl-N-ethylamin (Hünigbase) (aus N,N-Diisopropylamin und Acetaldehyd), N-Methyl-N-isopropylamin (MMIPA) (aus Monomethylamin und Aceton), n-Propylamine (wie Mono-/Di-n-propylamin, N,N-Dimethyl-N-n-propylamin (DMPA)) (aus Propionaldehyd und/oder n-Propanol und NH₃ bzw. DMA), N,N-Dimethyl-N-isopropylamin (DMIPA) (aus i-Propanol und/oder Aceton und DMA), N,N-Dimethyl-N-butylamine (1-, 2- oder iso-Butanol und/oder Butanal, i-Butanal oder Butanon und DMA), 2-(2-Di(C₁₋₄-alkyl)aminoethoxy)ethanol und/oder Bis(2-di(C₁₋₄-alkyl)aminoethyl)ether (aus DEG und Di(C₁₋₄-alkyl)amin), 1,2-Ethylendiamin (EDA), Monoethanolamin (MEOA), Diethylentriamin (DETA) und/oder Piperazin (PIP) (aus Monoethylenglykol (MEG) und Ammoniak), 1,8-Diamino-3,6-dioxa-octan und/oder 1-Amino-8-hydroxy-3,6-dioxa-octan (aus Triethylenglykol (TEG) und Ammoniak), 1-Methoxy-2-propylamin (1-Methoxy-isopropylamin, MOIPA) (aus 1-Methoxy-2-propanol und Ammoniak), N-Cyclododecyl-2,6-dimethylmorpholin (Dodemorph) (aus Cyclododecanon und/oder Cyclododecanol und 2,6-Dimethylmorpholin), Polyetheramin (aus entsprechendem Polyetheralkohol und Ammoniak). Die Polyetheralkohole sind z. B. Polyethylenglykole oder Polypropylenglykole mit einem Molekulargewicht im Bereich von 200 bis 5000 g/mol, die entsprechende Polyetheramine sind z. B. unter dem Handelsnamen PEA D230, D400, D2000, T403 oder T5000 von BASF erhältlich.

Besonders bevorzugt ist das Amin (A) mindestens ein Amin ausgewählt aus Monoamindiglykol (ADG), Morpholin, N-(C₁₋₄-alkyl)-Morpholin, 2-(2-Di(C₁₋₄-alkyl)-aminoethoxy)ethanol, Bis(2-di(C₁₋₄-alkyl)aminoethyl)ether, Monoethanolamin (MEOA), 1,2-Ethylendiamin (EDA), Polyetheraminen, Piperazin, Diethylentriamin (DETA) oder Polyisobutenamin (PIBA).

In einer bevorzugten Ausführungsform der vorliegenden Erfindung wird Monoaminodiklykol (ADG) und Morpholin durch Umsetzung von Diethylenglykol (DEG) mit Ammoniak hergestellt.

In einer weiteren bevorzugten Ausführungsform der vorliegenden Erfindung Wird N-(C₁₋₄-alkyl)morpholin durch Umsetzung von Diethylenglykol (DEG) mit Mono(C₁₋₄-alkyl)amin hergestellt.

In einer weiteren bevorzugten Ausführungsform der vorliegenden Erfindung wird 2-(2-Di(C₁₋₄-alkyl)aminoethoxy)ethanol und/oder Bis(2-di(C₁₋₄-alkyl)aminoethyl)ether durch Umsetzung von Diethylenglykol (DEG) mit Di(C₁₋₄-alkyl)amin hergestellt.

In einer weiteren bevorzugten Ausführungsform der vorliegenden Erfindung wird Monoethanolamin (MEOA) und/oder 1,2-Ethylendiamin (EDA) durch Umsetzung von Monoethylenglykol (MEG) mit Ammoniak hergestellt.

In einer weiteren bevorzugten Ausführungsform der vorliegenden Erfindung wird 1,2-Ethylendiamin (EDA) durch Umsetzung von Monoethanolamin (MEOA) mit Ammoniak hergestellt.

In einer weiteren bevorzugten Ausführungsform der vorliegenden Erfindung wird ein Polyetheramin durch Umsetzung eines entsprechenden Polyetheralkohols mit Ammoniak hergestellt.

In einer weiteren bevorzugten Ausführungsform der vorliegenden Erfindung wird Piperazin und/oder Diethylentriamin (DETA) durch Umsetzung von N-(2-Aminöethyl)-ethanolamin (AEEA) mit Ammoniak hergestellt.

In einer weiteren bevorzugten Ausführungsform der vorliegenden Erfindung wird Polyisobutenamin (PIBA) durch Umsetzung von Polyisobutenaldehyd mit Ammoniak hergestellt.

Nachfolgend wird die Erfindung anhand von Beispielen näher erläutert.

### Beispiele

### A) Herstellung der Lösungen

### A1) Metallsalzlösung (MSL1)

8,2 kg Nickelnitratlösung (13,5% Ni-Gehalt), 3,9kg Kupfernitratlösung (15,5% Cu-Gehalt) und 9,0 kg Cobaltnitratlösung (12,2 % Co-Gehalt) werden zur Lösung (MSL1) vereinigt und bei Raumtemperatur (RT) gelagert. Die Lösung (MSL1) ist bei Raumtemperatur tagelang stabil und bleibt klar.

### A2) Sn-Lösung kalt (SnL1)

3,3 kg 65-%ige Salpetersäure werden durch Zugabe von 4 kg Eis auf ca. 30 % verdünnt und bei diesem Lösevorgang auf etwa -15 °C abgekühlt. 100 g Zinnpulver werden zugesetzt, nach erfolgter Auflösung beträgt die Temperatur der Lösung etwa -5 °C. Die Lösung (SnL1) wird thermostatisiert bei 0 °C gehalten und bleibt so über mindestens einen Tag stabil und klar.

### A3) Sn-Lösung mit Citronensäure (SnL2)

Zu einem weiteren Ansatz der Lösung (SnL1) wird nach Auflösung des Zinns 354 g Citronensäure zugesetzt. Nach Erwärmung der Lösung auf Raumtemperatur bleibt die Lösung (SnL2) tagelang stabil und klar.

### A4) Sn-Lösung mit Citronensäure und Metallsalz (SnL3)

Lösung (SnL2) bei Raumtemperatur und Lösung (MSL1) werden vereinigt. Die Lösung (SnL3) bleibt tagelang stabil und klar.

### A5) Sn-Lösung mit Metallsalz bei 0 °C (SnL4)

Ein weiterer Ansatz der Lösung (MSL1) wird mittels eines Kryostaten auf 0 °C abgekühlt und mit einem weiteren Ansatz der Lösung (SnL1) bei 0 °C vereinigt. Die Lösung (SnL4) bleibt bei 0 °C über mindestens eine Tag stabil und klar.

### A6) Sn-Lösung mit Metallsalz bei RT (SnL5)

Weitere Ansätze gemäß Lösung (SnL1) (bei 0 °C) und Lösung (MSL1) (bei Raumtemperatur) werden zur Lösung (SnL5) vereinigt. Nach etwa einer Stunde ohne aktive Kühlung ist eine Trübung und beginnende Ausfällung erkennbar. Analytisch handelt es sich um einen Sn-haltigen Feststoff.

### A7) Sn-Lösung mit Metallsalz und Citronensäure (SnL6)

Zu 7,3 kg 30-%iger Salpetersäure werden bei Raumtemperatur 354 g Citronensäure zugesetzt. Anschließend werden bei Raumtemperatur 100 g Zinnpulver zugesetzt. Das Zinnpulver löst sich innerhalb weniger Minuten auf; die resultierende Lösung bleibt bei Raumtemperatur tagelang stabil und klar. Im Anschluss an die Stabilitätsprüfung wird diese Lösung bei Raumtemperatur mit einem weiteren Ansatz der Lösung (MSL1) unter Erhalt der Lösung (SnL6) vereinigt, die wiederum bei Raumtemperatur tagelang stabil und klar bleibt.

### B) Herstellung der Katalysatoren

### B1) Katalysator enthaltend 17,6 % Ni, 17,3 % Co, 9,7 % Cu, 0,9 % Sn, Rest Al₂O₃ (Vergleich)

Eine wässrige Lösung aus Nickelnitrat, Cobaltnitrat, Kupfernitrat, Zinnchlorid und fein dispergiertem Aluminiumoxidpulver (D10-10 von BASF SE), die rechnerisch jeweils 1,96 Gew.-% NiO und CoO, 0,94 % CuO, 0,09 % SnO und 3,76 % Al2O3 enthält, wird gleichzeitig in einem Rührgefäß in einem konstanten Strom mit einer 20 Gew.-%igen wässrigen Natriumcarbonatlösung bei einer Temperatur von 70 °C so gefällt, dass der mit einer Glaselektrode gemessene pH-Wert von 7,0 aufrechterhalten wurde. Die erhaltene Suspension wird filtriert und der Filterkuchen mit voll entsalztem Wasser gewaschen, bis die elektrische Leitfähigkeit des Filtrats ca. 20 µS beträgt. Danach wird der Filterkuchen bei einer Temperatur von 150 °C in einem Trockenschrank oder einem Sprühtrockner getrocknet. Das auf diese Weise erhaltene Hydroxid-Carbonat-Gemisch wird nun bei einer Temperatur von 450 bis 500 °C über einen Zeitraum von vier Stunden calciniert. Der so hergestellte Katalysator hat die Zusammensetzung: jeweils 23 Gew.-% NiO und CoO, 11 % CuO, 1 % SnO und 42 % Al2O3.

Der Katalysator wird mit 3 Gew.-% Graphit vermischt und zu Tabletten verformt. Die oxidischen Tabletten werden reduziert. Die Reduktion wird bei 280 °C durchgeführt, wobei die Aufheizungsrate 3 °C/Minute beträgt. Zuerst wird 50 Minuten mit 10 % H₂ in N₂ reduziert, dann 10 Minuten mit 75% H₂ in N₂ und schließlich 3 Stunden mit 100 % H₂. Bei den Prozentangaben handelt es sich um Volumenprozent. Die Passivierung des reduzierten Katalysators wird bei Raumtemperatur in verdünnter Luft (Luft in N₂ mit einem O₂-Gehalt von maximal 5 Vol.-%) durchgeführt.

Zusätzlich wird noch der Chlorgehalt zu 0,09 % bestimmt.

### B2) Katalysator auf Basis von Lösung (SnL5) / (Vergleich)

Zu einer Suspension aus 2,4 kg Al₂O₃ (D10-10 von der BASF, Glühverlust 12 %) in 7,5 kg H₂O wird gleichzeitig die komplette Lösung (SnL5) aus Beispiel A6 und wässrige 20%ige Natriumcarbonatlösung bei einer Temperatur von 65-70 °C so zugesetzt, dass ein mit einer Glaselektrode gemessener pH-Wert von 5,7+/-0,1 aufrechterhalten wird. Nach der Fällung wird für eine Stunde Luft eingeblasen, um gelöstes CO₂ zu entfernen, danach wird der pH der Suspension mit 20%iger Natriumcarbonatlösung auf den Wert 7,4 eingestellt. Die erhaltene Suspension wird filtriert und der Filterkuchen mit vollentsalztem Wasser gewaschen, bis die elektrische Leitfähigkeit des Filtrates ca. 500 microS beträgt. Danach wird der Filterkuchen bei einer Temperatur von 120 °C getrocknet. Das auf diese Weise erhaltene Hydroxidcarbonatgemisch wird nun in einem Drehkolbenofen bei einer Temperatur von 450 °C über 6 h unter Durchleiten von 200 l/h Luft/kg Pulver kalziniert. Anschließend wird das kalzinierte Pulver mit 3 % Graphit vermischt und zu Tabletten 3*3 mm mit einem Rüttelgewicht von 1300 g/l +/-100 g/l verformt. Die auf diese Weise erhaltenen Tabletten werden bei einer Temperatur von 280-300 °C in einem Stickstoff-/Wasserstoff-Gemisch (zu Beginn 1 % H₂ in N₂ bis zu 50 % H₂ in N₂) reduziert. Die Passivierung des reduzierten Katalysators wird im Anschluss bei maximal 35 °C in verdünnter Luft (Luft in N₂ mit einem O₂-Gehalt von zunächst 0,1 % bis maximal 10 Vol.-%) durchgeführt. Der so erhaltene Katalysator hat eine Zusammensetzung wie folgt: 18,8 % Ni, 19,2 % Co, 10,6 % Cu, 1,8 % Sn, Rest Al₂O₃. Der Katalysator enthält kein Chlor.

### B3 Katalysator auf Basis von Lösung (SnL6)

Man verfährt wie unter B2 beschrieben unter Einsatz der kompletten Lösung (SnL6) aus Beispiel A7. Der so erhaltene Katalysator hat eine Zusammensetzung wie folgt: 18,0 % Ni, 18,5 % Co, 10,1 % Cu, 1,7 % Sn, Rest Al₂O₃. Der Katalysator enthält kein Chlor.

### C) Katalysetests im kontinuierlich betriebenen Monoline-Reaktor

Ein beheizter Rohrreaktor, bestehend aus vier in Serie geschalteten Rohren mit jeweils 2,5 m Länge und einem Innendurchmesser von 4 mm wird mit etwa 80 g eines der oben beschriebenen Katalysatoren befüllt; der untere und obere Teil jeden Rohres (jeweils etwa 10-20 % des Rohrvolumens) wird mit einer Schicht Glaskugeln befüllt.

Vor der Reaktion wird der Katalysator bei 280 °C unter Durchleiten von 20 Nl/h Wasserstoff bei Normaldruck für 24 h aktiviert. Nach Absenkung der Temperatur auf etwa 200 °C und Erhöhung des Druckes auf 200 bar werden von unten nach oben pro Stunde Diethylenglykol (1,0 kg/l/h), Ammoniak (molares Verhältnis NH₃:DEG = 6) und Wasserstoff (molares Verhältnis H₂:DEG = 0,8) dosiert. Im Anschluss wird die Reaktionstemperatur so eingestellt, dass ein DEG-Umsatz von ca. 70 % erreicht wird. Das aus dem Reaktor austretende Gemisch wird abgekühlt und auf Normaldruck entspannt. Nach einer Einlaufphase von ca. 500 Stunden werden Proben vom Reaktionsgemisch (Flüssigaustrag) genommen und mittels Gaschromatographie analysiert. Die Ergebnisse gibt nachfolgende Tabelle wieder. Die in der nachfolgenden Tabelle 1 angegebenen Werte der Aminselektivität beziehen sich auf die Summe der beiden erhaltenen Reaktionsprodukte Monoaminodiglykol (ADG) und Morpholin.

**Tabelle 1**

| Beispiel | Katalysator | Temperatur | DEG-Umsatz mol% | Aminselektivität mol% |
|---|---|---|---|---|
| C1 | B1 - Vergleich | 205 | 69 | 91 |
| C2 | B2 - Vergleich | 203 | 70 | 90 |
| C3 | B3 - erfindungsgemäß | 205 | 69 | 92 |

Nach Ausbau des Vergleichskatalysators C1 wird nochmals der Chlorgehalt bestimmt; es kann eine Reduktion von 0,09 % auf 0,01 % festgestellt werden.

Das erfindungsgemäße Beispiel (C3) zeigt klar, dass durch die Stabilisierung der Sn-Lösung eine um 1-2 %-Punkte erhöhte Selektivität bei praktisch unveränderter Aktivität gegenüber Katalysatoren, die gemäß dem Stand der Technik hergestellt werden (C1) oder bei denen eine instabile Sn-Lösung eingesetzte wird (C2), erhalten werden kann. Darüber hinaus enthalten die erfindungsgemäßen Katalysatoren kein Chlor, was im Laufe der Reaktion verloren geht (siehe Beispiel C1) und so zu Korrosionsproblemen führt.

## Patentansprüche

1. Verfahren zur Herstellung eines geträgerten zinnhaltigen Katalysators, **dadurch gekennzeichnet, dass** eine Lösung (L) enthaltend Zinnnitrat und mindestens einen Komplexbildner auf den Träger aufgebracht wird, wobei die Lösung (L) keinen Feststoff oder einen Feststoffanteil von maximal 0,5 Gew.-% bezogen auf die Gesamtmenge an gelösten Komponenten enthält.

2. Verfahren gemäß Anspruch 1, **dadurch gekennzeichnet, dass** die Lösung (L) eine wässrige Lösung ist.

3. Verfahren gemäß Anspruch 1 oder 2, **dadurch gekennzeichnet, dass** die Lösung (L) zusätzlich mindestens ein weiteres Metallsalz enthält, vorzugsweise ein Nickelsalz, ein Cobaltsalz und/oder ein Kupfersalz enthält, besonders bevorzugt Nickelnitrat, Cobaltnitrat und/oder Kupfernitrat enthält.

4. Verfahren gemäß einem der Ansprüche 1 bis 3, **dadurch gekennzeichnet, dass** der Träger Aluminiumoxid ist.

5. Verfahren gemäß einem der Ansprüche 1 bis 4, **dadurch gekennzeichnet, dass** der Komplexbildner ausgewählt ist aus Glykolsäure, Milchsäure, Hydracylsäure, Hydroxybuttersäure, Hydroxyvaleriansäure, Äpfelsäure, Mandelsäure, Citronensäure, Zuckersäuren, Tartronsäure, Weinsäure, Oxalsäure, Malonsäure, Maleinsäure, Bernsteinsäure, Glutarsäure, Adipinsäure, Glycin, Hippursäure, EDTA, Alanin, Valin, Leucin oder Isoleucin.

6. Verfahren gemäß einem der Ansprüche 1 bis 5, **dadurch gekennzeichnet, dass** die Lösung (L) Zinnnitrat, Nickelnitrat, Cobaltnitrat, Kupfernitrat und Citronensäure enthält.

7. Verfahren gemäß einem der Ansprüche 1 bis 6, **dadurch gekennzeichnet, dass** die Lösung (L) hergestellt wird durch
i) Auflösen von Zinn in Salpetersäure bei einer Temperatur von maximal 5 °C und anschließender Zugabe von mindestens einem Komplexbildner, oder
ii) Auflösen von mindestens einem Komplexbildner in Salpetersäure bei einer Temperatur von 0 °C bis 50 °C, vorzugsweise bei Raumtemperatur, und anschließender Zugabe von Zinn.

8. Verfahren gemäß einem der Ansprüche 1 bis 7, **dadurch gekennzeichnet, dass** das Zinnnitrat, mindestens ein Komplexbildner und gegebenenfalls mindestens ein weiteres Metallsalz durch Zugabe von mindestens einer Base (B) aus der Lösung (L) auf den Träger ausgefällt werden, vorzugsweise durch Zugabe einer wässrigen Lösung der Base (B).

9. Verfahren gemäß Anspruch 8, **dadurch gekennzeichnet, dass** die Base (B) ausgewählt ist aus Natriumcarbonat, Natriumhydroxid, Kaliumcarbonat, Kaliumhydroxid oder alkalimetallfreien Basen wie Ammoniak, Ammoniumcarbonat, Ammoniumhydrogencarbonat, Ammoniumcarbamat, Ammoniumoxalat, Ammoniummalonat, Urotropin oder Harnstoff, vorzugsweise ist die Base (B) Natriumcarbonat.

10. Verfahren gemäß einem der Ansprüche 1 bis 9, **dadurch gekennzeichnet, dass** nach Aufbringen der Lösung (L) auf den Träger mindestens ein weiterer Schritt ausgewählt aus einem Trocknungsschritt, einem Calcinationsschritt, einem Konditionierungsschritt, einem Formgebungsschritt, einem Reduktionsschritt oder einem Passivierungsschritt durchgeführt wird.

11. Verfahren gemäß Anspruch 10, **dadurch gekennzeichnet, dass** der Komplexbildner während oder im Anschluss an den Calcinationsschritt aus dem geträgerten zinnhaltigen Katalysator entfernt wird.

12. Geträgerter zinnhaltiger Katalysator herstellbar nach einem Verfahren gemäß einem der Ansprüche 1 bis 11.

13. Katalysator gemäß Anspruch 12 enthaltend
i) 0,2 bis 5 Gew.-% Zinn,
ii) 15 bis 80 Gew.-% Aluminium,
iii) 1 bis 20 Gew.-% Kupfer,
iv) 5 bis 35 Gew.-% Nickel, und
v) 5 bis 35 Gew.-% Cobalt,
wobei die Gewichtsangaben der Komponenten i) bis v) als Oxide nach einem Calcinationsschritt und vor einem Reduktionsschritt mit Wasserstoff bestimmt werden.

14. Katalysator gemäß Anspruch 12 oder 13, **dadurch gekennzeichnet, dass** der Katalysator kein Chlor enthält.

15. Verfahren zur Herstellung eines Amins (A) in Gegenwart eines Katalysators gemäß einem der Ansprüche 12 bis 14.

16. Verfahren gemäß Anspruch 15, **dadurch gekennzeichnet, dass** ein primärer Alkohol, ein sekundärer Alkohol, ein Aldehyd und/oder ein Keton mit Wasserstoff und einer stickstoffhaltigen Verbindung (S) umgesetzt werden.

17. Verfahren gemäß Anspruch 16, **dadurch gekennzeichnet, dass** die stickstoffhaltige Verbindung (S) Ammoniak, ein primäres Amin und/oder ein sekundäres Amin ist.

18. Verfahren gemäß Anspruch 16 oder 17, **dadurch gekennzeichnet, dass** der primäre Alkohol, der sekundäre Alkohol, das Aldehyd, das Keton und/oder die stickstoffhaltige Verbindung (S) als wässrige Lösung eingesetzt werden.

19. Verfahren gemäß einem der Ansprüche 15 bis 18, **dadurch gekennzeichnet, dass** das Amin (A) mindestens ein Amin ausgewählt aus Monoamindiglykol (ADG), Morpholin, N-(C₁₋₄-alkyl)-Morpholin, 2-(2-Di(C₁₋₄-alkyl)aminoethoxy)ethanol, Bis(2-di(C₁₋₄-alkyl)aminoethyl)ether, Monoethanolamin (MEOA), 1,2-Ethylendiamin (EDA), Polyetheraminen, Piperazin, Diethylentriamin (DETA) oder Polyisobutenamin (PIBA) ist.

20. Verfahren gemäß einem der Ansprüche 15 bis 19, **dadurch gekennzeichnet, dass** Monoaminodiklykol (ADG) und Morpholin durch Umsetzung von Diethylenglykol (DEG) mit Ammoniak hergestellt werden.

## Claims

1. A process for producing a supported tin-comprising catalyst, wherein a solution (S) comprising tin nitrate and at least one complexing agent is applied to the support, where the solution (S) does not comprise any solid or comprises a solids content of not more than 0.5% by weight based on the total amount of dissolved components.

2. The process according to claim 1, wherein the solution (S) is an aqueous solution.

3. The process according to claim 1 or 2, wherein the solution (S) additionally comprises at least one further metal salt, preferably a nickel salt, a cobalt salt and/or a copper salt, particularly preferably nickel nitrate, cobalt nitrate and/or copper nitrate.

4. The process according to any of claims 1 to 3, wherein the support is aluminum oxide.

5. The process according to any of claims 1 to 4, wherein the complexing agent is selected from among glycolic acid, lactic acid, hydracylic acid, hydroxybutyric acid, hydroxyvaleric acid, malic acid, mandelic acid, citric acid, sugar acids, tartronic acid, tartaric acid, oxalic acid, malonic acid, maleic acid, succinic acid, glutaric acid, adipic acid, glycine, hippuric acid, EDTA, alanine, valine, leucine and isoleucine.

6. The process according to any of claims 1 to 5, wherein the solution (S) comprises tin nitrate, nickel nitrate, cobalt nitrate, copper nitrate and citric acid.

7. The process according to any of claims 1 to 6, wherein the solution (S) is produced by
i) dissolving tin in nitric acid at a temperature of not more than 5°C and subsequently adding at least one complexing agent, or
ii) dissolving at least one complexing agent in nitric acid at a temperature of from 0°C to 50°C, preferably at room temperature, and subsequently adding tin.

8. The process according to any of claims 1 to 7, wherein the tin nitrate, at least one complexing agent and optionally at least one further metal salt are precipitated from the solution (S) onto the support by addition of at least one base (B), preferably by addition of an aqueous solution of the base (B).

9. The process according to claim 8, wherein the base (B) is selected from among sodium carbonate, sodium hydroxide, potassium carbonate, potassium hydroxide and bases which are free of alkali metals, for example ammonia, ammonium carbonate, ammonium hydrogencarbonate, ammonium carbamate, ammonium oxalate, ammonium malonate, urotropin or urea, with preference being given to the base (B) being sodium carbonate.

10. The process according to any of claims 1 to 9, wherein application of the solution (S) to the support is followed by at least one further step selected from among a drying step, a calcination step, a conditioning step, a shaping step, a reduction step and a passivation step.

11. The process according to claim 10, wherein the complexing agent is removed from the supported tin-comprising catalyst during or after the calcination step.

12. A supported tin-comprising catalyst which can be produced by a process according to any of claims 1 to 11.

13. The catalyst according to claim 12 comprising
i) from 0.2 to 5% by weight of tin,
ii) from 15 to 80% by weight of aluminum,
iii) from 1 to 20% by weight of copper,
iv) from 5 to 35% by weight of nickel and
v) from 5 to 35% by weight of cobalt,
where the proportions by weight of the components i) to v) are determined as oxides after a calcination step and before a reduction step using hydrogen.

14. The catalyst according to claim 12 or 13, wherein the catalyst does not comprise any chlorine.

15. A process for preparing an amine (A) in the presence of a catalyst according to any of claims 12 to 14.

16. The process according to claim 15, wherein a primary alcohol, a secondary alcohol, an aldehyde and/or a ketone is/are reacted with hydrogen and a nitrogen-comprising compound (N).

17. The process according to claim 16, wherein the nitrogen-comprising compound (N) is ammonia, a primary amine and/or a secondary amine.

18. The process according to claim 16 or 17, wherein the primary alcohol, the secondary alcohol, the aldehyde, the ketone and/or the nitrogen-comprising compound (N) is/are used as aqueous solution.

19. The process according to any of claims 15 to 18, wherein the amine (A) is at least one amine selected from among monoaminodiglycol (ADG), morpholine, N-(C₁₋₄-alkyl)morpholine, 2-(2-di(C₁₋₄-alkyl)aminoethoxy)ethanol, bis(2-di(C₁₋₄-alkyl)aminoethyl) ether, monoethanolamine (MEOA), 1,2-ethylenediamine (EDA), polyetheramines, piperazine, diethylenetriamine (DETA) and polyisobutenamine (PIBA).

20. The process according to any of claims 15 to 19, wherein monoaminodiglycol (ADG) and morpholine are prepared by reacting diethylene glycol (DEG) with ammonia.

## Revendications

1. Procédé de fabrication d'un catalyseur supporté contenant de l'étain, **caractérisé en ce qu'**une solution (L) contenant du nitrate d'étain et au moins un complexant est appliquée sur le support, la solution (L) ne contenant pas de solides ou ayant une teneur en solides d'au plus 0,5 % en poids, par rapport à la quantité totale de composants dissous.

2. Procédé selon la revendication 1, **caractérisé en ce que** la solution (L) est une solution aqueuse.

3. Procédé selon la revendication 1 ou 2, **caractérisé en ce que** la solution (L) contient en outre au moins un sel métallique supplémentaire, de préférence un sel de nickel, un sel de cobalt et/ou un sel de cuivre, de manière particulièrement préférée du nitrate de nickel, du nitrate de cobalt et/ou du nitrate de cuivre.

4. Procédé selon l'une quelconque des revendications 1 à 3, **caractérisé en ce que** le support est l'oxyde d'aluminium.

5. Procédé selon l'une quelconque des revendications 1 à 4, **caractérisé en ce que** le complexant est choisi parmi l'acide glycolique, l'acide lactique, l'acide hydracylique, l'acide hydroxybutyrique, l'acide hydroxyvalérique, l'acide malique, l'acide mandélique, l'acide citrique, l'acide saccharique, l'acide tartronique, l'acide tartrique, l'acide oxalique, l'acide malonique, l'acide maléique, l'acide succinique, l'acide glutarique, l'acide adipique, la glycine, l'acide hippurique, l'EDTA, l'alanine, la valine, la leucine ou l'isoleucine.

6. Procédé selon l'une quelconque des revendications 1 à 5, **caractérisé en ce que** la solution (L) contient du nitrate d'étain, du nitrate de nickel, du nitrate de cobalt, du nitrate de cuivre et de l'acide citrique.

7. Procédé selon l'une quelconque des revendications 1 à 6, **caractérisé en ce que** la solution (L) est fabriquée par
i) dissolution d'étain dans de l'acide nitrique à une température d'au plus 5 °C, puis ajout d'au moins un complexant, ou
ii) dissolution d'au moins un complexant dans de l'acide nitrique à une température de 0 °C à 50 °C, de préférence à température ambiante, puis ajout d'étain.

8. Procédé selon l'une quelconque des revendications 1 à 7, **caractérisé en ce que** le nitrate d'étain, au moins un complexant et éventuellement au moins un sel métallique supplémentaire sont précipités sur le support à partir de la solution (L) par ajout d'au moins une base (B), de préférence par ajout d'une solution aqueuse de la base (B).

9. Procédé selon la revendication 8, **caractérisé en ce que** la base (B) est choisie parmi le carbonate de sodium, l'hydroxyde de sodium, le carbonate de potassium, l'hydroxyde de potassium ou les bases sans métaux alcalins, telles que l'ammoniac, le carbonate d'ammonium, l'hydrogénocarbonate d'ammonium, le carbamate d'ammonium, l'oxalate d'ammonium, le malonate d'ammonium, l'urotropine ou l'urée, la base (B) étant de préférence le carbonate de sodium.

10. Procédé selon l'une quelconque des revendications 1 à 9, **caractérisé en ce qu'**au moins une étape supplémentaire choisie parmi une étape de séchage, une étape de calcination, une étape de conditionnement, une étape de façonnage, une étape de réduction ou une étape de passivation est réalisée après l'application de la solution (L) sur le support.

11. Procédé selon la revendication 10, **caractérisé en ce que** le complexant est éliminé du catalyseur supporté contenant de l'étain pendant ou après l'étape de calcination.

12. Catalyseur supporté contenant de l'étain, pouvant être fabriqué par un procédé selon l'une quelconque des revendications 1 à 11.

13. Catalyseur selon la revendication 12, contenant :
i) 0,2 à 5 % en poids d'étain,
ii) 15 à 80 % en poids d'aluminium,
iii) 1 à 20 % en poids de cuivre,
iv) 5 à 35 % en poids de nickel, et
v) 5 à 35 % en poids de cobalt,
les données en poids des composants i) à v) étant déterminées sous la forme d'oxydes après une étape de calcination et avant une étape de réduction avec de l'hydrogène.

14. Catalyseur selon la revendication 12 ou 13, **caractérisé en ce que** le catalyseur ne contient pas de chlore.

15. Procédé de fabrication d'une amine (A) en présence d'un catalyseur selon l'une quelconque des revendications 12 à 14.

16. Procédé selon la revendication 15, **caractérisé en ce qu'**un alcool primaire, un alcool secondaire, un aldéhyde et/ou une cétone sont mis en réaction avec de l'hydrogène et un composé azoté (S).

17. Procédé selon la revendication 16, **caractérisé en ce que** le composé azoté (S) est l'ammoniac, une amine primaire et/ou une amine secondaire.

18. Procédé selon la revendication 16 ou 17, **caractérisé en ce que** l'alcool primaire, l'alcool secondaire, l'aldéhyde, la cétone et/ou le composé azoté (S) sont utilisés sous la forme d'une solution aqueuse.

19. Procédé selon l'une quelconque des revendications 15 à 18, **caractérisé en ce que** l'amine (A) est au moins une amine choisie parmi le monoaminodiglycol (ADG), la morpholine, la N-(alkyle en C₁₋₄)-morpholine, le 2-(2-di (alkyle en C₁₋₄)aminoéthoxy)éthanol, l'éther bis(2-di(alkyle en C₁₋₄)aminoéthylique), la monoéthanolamine (MEOA), la 1,2-éthylène-diamine (EDA), les ρolyétheramines, la pipérazine, la diéthylène-triamine (DETA) ou la polyisobutène-amine (PIBA).

20. Procédé selon l'une quelconque des revendications 15 à 19, **caractérisé en ce que** du monoaminodiglycol (ADG) et de la morpholine sont fabriqués par mise en réaction de diéthylène glycol (DEG) avec de l'ammoniac.
